# EUROPEAN PATENT SPECIFICATION

(11) **EP 1 370 288 B1**
(45) Date of publication and mention of the grant of the patent: **11.01.2012**
(21) Application number: 01996390.9
(22) Date of filing: 20.11.2001
(51) Int. Cl.: A61K 39/395, A61P 7/00, C07K 16/28

(54) **METHOD FOR TREATING THROMBOCYTOPENIA WITH MONOCLONAL IVIG**
VERFAHREN ZUR BEHANDLUNG DER THROMBOZYTOPENIE MIT MONOKLONALEM IVIG
PROCEDE SERVANT A TRAITER LA THROMBOCYTOPENIE AU MOYEN D'IMMUNOGLOBULINE INTRAVEINEUSE MONOCLONALE

(30) Priority: 20.11.2000 US 249441 P
(43) Date of publication of application: 17.12.2003
(60) Divisional application: 10178480.9
(73) Proprietor: Canadian Blood Services, Ottawa, Ontario K1G 4J5 (CA)
(72) Inventor: LAZARUS, Alan, H., Toronto, Ontario M4H 1J6 (CA); FREEDMAN, John, Toronto, Ontario M4Y 1R8 (CA); SONG, Seng, Toronto, Ontario M4C 4Y2 (CA); CROW, Andrew, R., Toronto, Ontario M4C 5L4 (CA)
(74) Representative: Williams, Aylsa
(86) International application number: PCT/CA2001/001648
(87) International publication number: WO 2002/040047

(56) References cited:
- WO-A-99/00143
- WO-A-99/03495
- WO-A-99/03495
- CA-A1- 2 372 603
- WALLACE P K ET AL: "HUMANIZED MAB H22 BINDS THE HUMAN HIGH AFFINITY FC RECEPTOR FOR IGG (FCGAMMARI), BLOCKS PHAGOCYTOSIS, AND MODULATES RECEPTOR EXPRESSION" JOURNAL OF LEUKOCYTE BIOLOGY, FEDERATION OF AMERICAN SOCIETIES FOR EXPERIMENTAL, US, vol. 62, no. 4, October 1997 (1997-10), pages 469-479, XP008011544 ISSN: 0741-5400
- DATABASE WPI Section Ch, Week 199723 Derwent Publications Ltd., London, GB; Class B04, AN 1997-255474 XP002374401 & JP 09 087202 A (SUMITOMO SEIYAKU KK) 31 March 1997 (1997-03-31)
- LAZARUS ALAN H ET AL: "Monoclonal antibodies which mimic the action of intravenous immunoglobulin (mIVIG) can inhibit immune thrombocytopenia" BLOOD, vol. 98, no. 11 Part 1, 16 November 2001 (2001-11-16), page 441a, XP002374393 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971
- SONG SENG ET AL: "Monoclonal IgG can ameliorate immune thrombocytopenia in a murine model of ITP: An alternative to IVIG." BLOOD, vol. 101, no. 9, May 2003 (2003-05), pages 3708-3713, XP002374394 ISSN: 0006-4971
- BUSSEL J B: "Fc receptor blockade and immune thrombocytopenic purpura." SEMINARS IN HEMATOLOGY. UNITED STATES JUL 2000, vol. 37, no. 3, July 2000 (2000-07), pages 261-266, XP008011545 ISSN: 0037-1963
- DATABASE MEDLINE [Online] November 1989 (1989-11) BUSSEL J B: "The use of intravenous gamma-globulin in idiopathic thrombocytopenic purpura." Database accession no. NLM2477185 XP002226230 & CLINICAL IMMUNOLOGY AND IMMUNOPATHOLOGY. UNITED STATES NOV 1989, vol. 53, no. 2 Pt 2, November 1989 (1989-11), pages S147-S155, ISSN: 0090-1229
- BUSSEL J B: "FC RECEPTOR BLOCKADE AND IMMUNE THROMBOCYTOPENIC PURPURA", SEMINARS IN HEMATOLOGY, PHILADELPHIA, PA, US, vol. 37, no. 3, 1 July 2000 (2000-07-01), pages 261-266, XP008011545, ISSN: 0037-1963

## Description

### BACKGROUND OF THE INVENTION

### (a) Field of the Invention

The invention relates to a composition for use in treating thrombocytopenia in a mammal.

### (b) Description of Prior Art

Intravenous immunoglobulin (IVIG) in therapeutic use is made from large numbers of human sera (therefore polyclonal) and is often in short supply. Although the product is considered to be safe by proponents, there is public concern.

IVIG is prepared from large pools of plasma from more than 10,000 normal healthy donors. Most preparations only contain IgG with minimal levels of "contaminants" such as IgA. The IgG is present in predominantly monomeric form, with a subclass distribution characteristic of the subclass distribution in normal serum. The first use of IVIG in treating idiopathic thrombocytopenic purpura (ITP) was in 1981 when high doses of IVIG were reported to promote fast recovery of ITP in children. Despite extensive clinical use, the mechanism of action of IVIG remains incompletely understood and even the threshold effective dose of IVIG remains poorly defined. Several theories have been proposed to explain how administration of IVIG to individuals with ITP reverses the platelet count. Following is an overview of some of the major theories.

### Reticuloendothelial system (RES) blockade.

It was initially postulated that the success of IVIG in treating ITP was due to competitive inhibition of the reticuloendothelial system (RES) by sensitized erythrocytes. The major site of platelet destruction in ITP is well known to involve the spleen. The spleen contains large numbers of Fc receptor-bearing phagocytic cells, such as monocytes and macrophages, which can bind and destroy opsonized platelets. Although the spleen may not be the only site of platelet destruction, splenectomy is a successful treatment for some individuals with ITP. Perhaps the most direct early evidence that RES blockade by IVIG can prolong the half-life of antibody-sensitized cells were experiments by Fehr and co-workers (Fehr et al., New Engl. J. Med., 306:1254-1258, 1982). Fehr studied four patients with ITP who had not undergone splenectomy. Infusion of IVIG prolonged the in vivo clearance of radiolabelled antibody-sensitized erythrocytes. These results have been confirmed by others, using both erythrocytes and platelets as target cells. Several studies comparing intact IVIG to F(ab')₂, fragments from IVIG (the latter do not bind to Fc receptors) have clearly indicated that the intact preparations are more efficacious in reversing the thrombocytopenia.

The spleen, and more generally the RES, possesses 3 classes of Fcγ receptors (R); Fcγ RI which binds non-complexed IgG, and Fcγ RII and Fcγ RIII, which both bind complexed IgG. Blocking the Fcγ RI seems to have no effect in ITP, whereas Clarkson and co-workers (Clarkson et al., New Engl. J. Med., 314(19):1236-1239, 1986) showed in a case report that an antibody specific for the Fcγ RIII, but also with specificity for Fcγ RII, ameliorated refractory thrombocytopenia. A study in animals has shown that administration of a monoclonal antibody (2.462) which specifically binds to the Fcγ RII within the RES can dramatically prevent clearance of IgG sensitized erythrocytes; whether or not this anti-Fc receptor antibody was able to ameliorate thrombocytopenia in ITP was not studied.

An example of an antigen-specific intravenous IgG preparation (and therefore an IVIG) is anti-D. The major mechanism of action proposed for anti-D is via RES blockade. This has been demonstrated by studies showing that anti-D appears to be ineffective in most patients who are Rh D antigen negative. In one study, 3 D negative, but c antigen positive, patients refractory to treatment with anti-D, were successfully treated with anti-c. Thus, an antibody which reacts with erythrocytes (whether to the D or c antigen, or likely any other appropriate antigen) can block the RES and increase the platelet count in ITP. A single small prospective study on 7 D positive patients with chronic ITP was initiated to test a human (IgG,) monoclonal anti-D (Godeau B, et al., Transfusion 36:328-330, 1996). This monoclonal antibody did not significantly ameliorate ITP. Subsequent to this finding which teaches that monoclonal anti-D cannot ameliorate thrombocytopenia several commentaries have also concluded that monoclonal IVIGs, and especially anti-red cell monoclonal antibodies or anti-D reagents are not even worthwhile considering (Atrah HI, Transfusion, 37:444, 1997; Neppert J, et al., Transfusion, 37:444-445, 1997; Godeau B, & Bierling P., Transfusion, 37:445-446, 1997; International forum: Engelfriet CP, Reesink HW, Bussel J, Godeau B, Bierling P, Panser S, Grumayer-Panzer ER, Mayer WR, Neppert J, Taaning E, Minchinton R. Bowman JM. The treatment of patients with autoimmune thrombocytopenia with intravenous IgG-anti-D. Vox Sang, 76:250-255, 1999).

RES blockade is generally the most highly accepted mechanism to account for the effects of IVIG. However, some patients do respond to F(ab')₂ fragments of IVIG and some are protected by IVIG but have a functional RES.

### Antiidiotypic antibodies

An alternative mechanism involves the regulatory properties of a subset of antibodies called antiidiotypic antibodies i.e. antibodies which bind to the antigen-combining region of other antibodies. The antigen-combining region of IgG contains the idiotypic region (hypervariable region) and possesses amino acid sequences that encode the fine specificity of each antibody. When an individual is immunized and produces antibodies against an antigen, for example ovalbumin, the resulting anti-ovalbumin antibody will possess an idiotypic region that has never before been seen by the host. In effect, exposure of a host to a foreign antigen results in the production of a new IgM or IgG molecule, which in turn possesses an idiotypic region foreign to the host. In the above example, the host responds by making IgM and IgG antibodies to the anti-ovalbumin antibody, these antibodies interact, form an immune complex, and the final effect is the neutralization of the antibodies.

One of the major targets of the autoantibodies in ITP is the platelet membrane glycoprotein (GP) IIbIIIa. Berchtold and co-workers (Berchtold, P., et al., Blood, 74, 2414-2417, 1989) demonstrated that therapeutic preparations of IVIG contain antibodies which can interact and neutralize the effects of anti-GPIIbIIIa and it was indirectly observed that platelet-reactive autoantibodies from patients with ITP can bind IVIG as assessed by a phage display method. This effect would prevent new platelets from encountering anti-GPIIbIIIa autoantibodies, which would be evidenced by a decrease in platelet-associated IgG and reversal of thrombocytopenia. A strong argument against antiidiotypic antibodies mediating the only effect in ITP is a study of 12 children with acute ITP who were treated with Fc fragments generated from IVIG; 11/12 showed a rapid rise in platelet count. Since Fc fragments from IVIG do not possess the antiidiotypic region of IgG, it is difficult to conclude that the reversal of thrombocytopenia observed was due to any antiidiotypic effect.

### Other mechanisms

IVIG has been demonstrated in a number of studies to have effects on the cellular immune response itself. Specifically, long term responses following IVIG administration have been shown to be associated with enhanced suppressor T lymphocyte function and decreased autoantibody production. IVIG has been shown to possess an unusual anti-FcεRIα reactivity. In one study, IVIG was shown to reduce the number of CD4+ T helper cells in vivo in some patients. IVIG prepared as monomeric or aggregated human gamma globulin has been shown to be capable of inducing immune tolerance in both B cells and T cells. The mechanism(s) of how IVIG exerts its regulatory functions on T cells has not yet been definitively established, but IVIG has been shown to affect both cytokine and cytokine receptor levels both in vitro and in vivo.

One of the in vitro effects of IVIG is the growth arrest of fibroblasts, hematopoetic cell lines, lymphocytes and endothelial cells. It was also demonstrated that some of the anti-proliferative effects of IVIG-induced growth arrest may be mediated by anti-glycolipid antibodies. There are other activities of IVIG described, such as the ability of IVIG to inhibit complement-dependent in vivo clearance of appropriately sensitized cells, to activate complement and promote complement-dependent RES sequestration of erythrocytes. IVIG has also been shown to affect apoptosis via a Fas-dependent effect. In addition, IVIG represents the antibody repertoire of a large number of individuals, all of whom would have been exposed to a variety of pathogens: this may have particular relevance for its success in acute childhood ITP, often associated with viral infection.

Finally, it is possible that some immune modulatory effects of IVIG are not due to the immunoglobulin fraction itself but are due to "contaminating" products present therein, including T cell growth factor, or TGF-β.

In summary, several contrasting mechanisms have been proposed to explain the rapid and long-term effects of IVIG. While the precise mechanism(s) of action of IVIG may be difficult to define, it is likely that many of the above mechanisms are not mutually exclusive and may contribute differentially or additively to the success of IVIG therapy. Although several studies have attempted to determine or purify the active component of IVIG (i.e. anti-self antibodies, antibody dimers, antiidiotypes against GPIIbIIIa, anti-glycolipid antibodies, etc) the ability of these IVIG components to reverse thrombocytopenia has not been examined.

Brussel J. B. (Seminars in Hematology (Vol 37) No. 5 pp 261-266 (July 2000)) is a review article which summarises that FcR blockade is an important mechanism of treatment effect in patients with auto-immune thrombocytopenic purpura (ITP).

It would be highly desirable to be provided with antibodies free of contaminants such as TGF-p.

It would also be highly desirable to be provided with a method for treating thrombocytopenia using monoclonal preparations.

### SUMMARY OF THE INVENTION

One aim of the present invention is to provide a pharmaceutical composition for use in treating a disease known as auto-immune thrombocytopenic purpura (ITP) with a "synthetically produced" monoclonal preparation of IVIG (mIVIG).

In accordance with the present invention there is provided a pharmaceutical composition for use in treating thrombocytopenia in a mammal, comprising an effective amount of a monoclonal antibody specific for blood cells in combination with a pharmaceutical acceptable carrier, wherein the monoclonal antibody is an anti-red blood cell antibody and is capable of inhibiting Reticuloendothelial System (RES). The anti-red blood cell antibody can be anti-CD24 or anti-TER-119.

The monoclonal antibody is preferably adapted for intravenous, interperitoneal, intramuscular or subcutaneous administration.

Also in accordance with the present invention, there is provided use of a pharmaceutical composition as defined above, comprising at least 1 µg of the monoclonal antibody per kg of body weight of the mammal, in the manufacture of a medicament for increasing the level of platelets in a mammal experiencing thrombocytopenia.

Still in accordance with the present invention, there is provided a pharmaceutical composition as defined above, comprising at least 1µg of the monoclonal antibody per kg of body weight of the mammal, for increasing the level of platelets in a mammal experiencing thrombocytopenia.

The therapeutic amount of the monoclonal intravenous immunoglobulin (mIVIG) administered preferably ranges from about 1 µg to about 1g per kg of body weight per day.

The pharmaceutical composition may further comprise human IVIG.

For the purpose of the present invention the following expressions or terms are defined below.

The expression direct blocking or direct Fc receptor blocking in this patent application is intended to mean, an antibody which has hypervariable regions that impart a distinct specificity for the Fcγ receptor (such as the FcγR II) and this antibody binds to the Fcγ receptor using these hypervariable regions.

The expression indirect blocking or indirect Fc receptor blocking in this patent application is intended to mean, an antibody which has hypervariable regions that impart a specificity for an antigen other than an an Fcγ receptor (such as the FcγR II) and this antibody binds to this other antigen forming an immune complex. The immune complex then would inhibit the reticular endothelial system by binding to a receptor or cell protein involved in platelet clearance. These receptors or cell proteins may include, but are not limited to any class of an Fc receptor, complement receptors, scavenger receptors, integrins, selectins, or their receptors etc.

The term immune complex in this patent application is intended to mean, an antibody which binds to at least one an antigen, including other antibodies.

### BRIEF DESCRIPTION OF THE DRAWINGS

Figs. 1A and 1 B illustrate the effect of Anti-platelet "auto"-antibody causing passive-immune thrombocytopenic purpura (P-ITP) in a dose dependent fashion on SCID mice injected with the indicated amount of anti-GPIIb (Fig. 1A) or anti-GPIIIa (Fig. 1B);
Figs. 2a and 2B illustrate the effects of anti-GPIIb (Fig. 2A) and anti-GPIIIa (Fig. 2B) on P-ITP;
Fig. 3 illustrates that standard human IVIG ameliorates P-ITP in a dose dependent manner in the mice which were unmanipulated (-----), or injected with anti-GPIIb (- - - -), or were therapeutically treated with the corresponding dose of IVIG and injected with anti -GPIIb (O-O);
Figs. 4a to 4D illustrate the effect of IgG₁ (Fig. 4A), IgG₂ₐ (Fig.
   4B), IgG_{2b} (Fig. 4C), and IgG_{2c} (Fig. 4D) on the platelet concentration of unmanipulated mice (column A), and mice injected with anti-GPIIb;
Fig. 5 illustrates that monoclonal IVIG (mIVIG) with specificity for the CD24 antigen on erythrocytes can ameliorate P-ITP;
Fig. 6 illustrates that monoclonal IVIG (mIVIG) with specificity for the TER-119 antigen (Ly-76) on erythrocytes can ameliorate P-ITP;
Fig. 7 illustrates that monoclonal antibody with specificity for the murine FcγRII (CD32) & RIII (CD16) receptors can ameliorate P-ITP;
Fig. 8 illustrates that monoclonal IVIG (mIVIG) with specificity for the CD44 antigen can ameliorate P-ITP; and
Fig. 9 illustrates a reticuloendothelial system (RES) blockade assay by mIVIG.

### DETAILED DESCRIPTION OF THE INVENTION

To determine if a monoclonal preparation of intravenous immunoglobulin of the IgG class (mIVIG) can ameliorate thrombocytopenia, mice were treated with mIVIG via tail vein injection followed by antibody-induced induction of thrombocytopenia. The mIVIG preparations directed against erythrocytes are shown to ameliorate thrombocytopenia. Although antibody reagents against Fc receptors would not likely be useful mIVIGs to treat thrombocytopenia due to these mIVIG directly binding cells in the RES by the variable region of the mIVIG, these reagents nevertheless can be useful, in general, to determine the potential mechanism of the mIVIG action in amelioration of thrombocytopenia.

To determine if reticuloendothelial system (RES) blockade was a likely mechanism of this affect, an antibody with specificity for the FcγRII and FcγRIII was examined. This antibody with specificity for the FcγRII and FcγRIII ameliorated thrombocytopenia. In the present invention, any mIVIG which can bind an *in vivo* antigen, and particularly those that block RES function but do not bind to FcRs directly, will be useful as a means to ameliorate thrombocytopenia. To demonstrate that antigens other than erythrocyte antigens can serve as mIVIG targets, an mIVIG which binds the CD44 antigen (not found on erythrocytes but found on most other cells) was also examined. This mIVIG also ameliorated thrombocytopenia.

SCID mice were injected intravenously with either 2 g IVIG /Kg body weight, 2 g/Kg control protein (albumin) or mIVIG. The mIVIG`s were all administered at doses of 2,000 µg/Kg, 200 µg/Kg and 20 µg/Kg. Mice were then returned to their cages for 24 h and then injected intraperitoneally with either 2 µg anti-GPIIb (purified antibody, clone: MWReg30) or 10 µg anti-GPIIIa (purified antibody, clone: 2C9.G2) antibodies in a total volume of 200 µl in PBS to induce passive-immune thrombocytopenic purpura (P-ITP). After 24 hr, 100 µl of blood was collected from the tail vein, immediately added to 400 µl of 1 % EDTA in PBS to prevent clotting and 5 µl added to a known amount of buffer (final dilution of blood:1/12 000) and analyzed by a calibrated flow cytometry. The events acquired in 2 minutes were used to calculate the number of platelets. Platelet gates were set based on forward and side scatter and by staining with a fluorescent platelet-specific antibody, essentially as described (Moody M, et al., Transfusion Med, 9:147-154, 1999).

### RESULTS

### Establishment of an in vivo mouse model of passive-immune thrombocytopenic purpura (P-ITP).

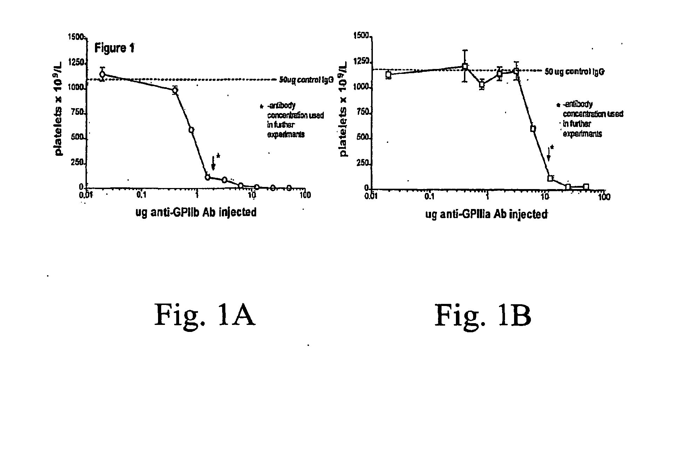

CD17 SCID female virgin mice were obtained from Charles River Labs (Montreal, PQ) and housed under gnotobiotic conditions. P-ITP was induced by injection of mice with monoclonal anti-platelet antibody (2 µg rat anti-mouse GPIIb, IgG₁, κ Pharmingen, Mississauga, ON) in 200 µl PBS pH 7.2. Twenty four hours later, whole blood was collected via the tail vein. The mice injected with different doses of either anti-GPIIb or anti-GPIIIa monoclonal antibodies exhibited a marked decrease in platelet counts as measured at 24 hours post injection (see Figs. 1A and 1B). The mice receiving control IgG did not exhibit any signs of thrombocytopenia (Figs. 1A and 1B, dotted line, n=5 mice). The mice receiving increasing amounts of anti-GPIIb (Fig. 1A) or increasing amounts of anti-GPIIIa (Fig. 1B) developed more severe thrombocytopenia. The dose corresponding to the arrow in both Figs. is the dose selected of each antibody which was used for subsequent experiments to induce thrombocytopenia or P-ITP. This dose was 2 µg/mouse for the anti-GPIIb antibody and 10 µg/mouse for the anti-GPIIIa antibody. The monoclonal hamster antimouse GPIIIa and rat antimouse GPIIb which were used to induce thrombocytopenia were purchased from Pharmingen (Mississauga, ON).

In Fig. 1A and 1B, each data point represents the mean in vivo platelet concentration (from whole blood) of 5 mice sampled 24 h post P-ITP induction. The mean platelet concentration of SCID mice injected with 50 µg/mouse of "non-specific" rat IgG is indicated by the dashed line.

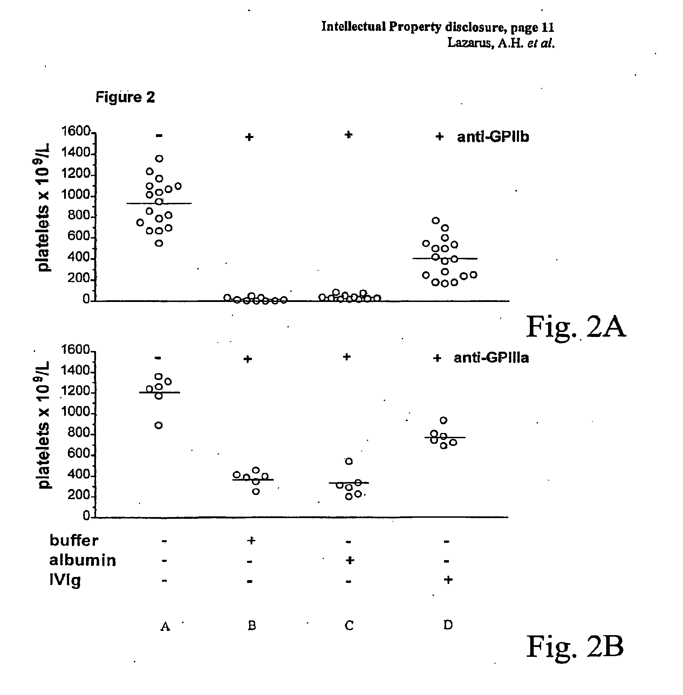

In the IVIg studies, mice were treated with the specified amount of IVIg (Gamimune N 5%, Bayer, Inc., Elkhart, IN), 24 hours prior to induction of P-ITP. Control mice were pretreated with an equivalent amount of ovalbumin in 10% maltose/PBS buffer alone. To determine if a standard commercial preparation of IVIG could protect mice from P-ITP-induced thrombocytopenia, the mice were injected with either buffer (PBS), 2 g/Kg human albumin, or 2g/Kg IVIG, followed by either anti-GPIIb (Fig. 2A) or anti-GPIIIa (Fig. 2B), as indicated. In both cases, 2 g/Kg IVIG protected against severe thrombocytopenia. Human-IVIG (Gamimune Bayer, Elkhart, IN) was used.

In Figs. 2A and 2B, it is shown that standard human IVIG ameliorates P-ITP. In Figs. 2A and 2B, column A represents unmanipulated mice, columns B-D represent mice injected with anti-GPIIb or anti-GPIIIa. In column B, mice were pretreated with PBS buffer. In column C, mice were pretreated with 2 g human serum albumin /Kg body weight. In column D, mice were therapeutically pretreated with 2 g IVIG /Kg body weight. Each data point represents the in vivo platelet concentration (from whole blood) of 1 mouse sampled 24 h post P-ITP induction.

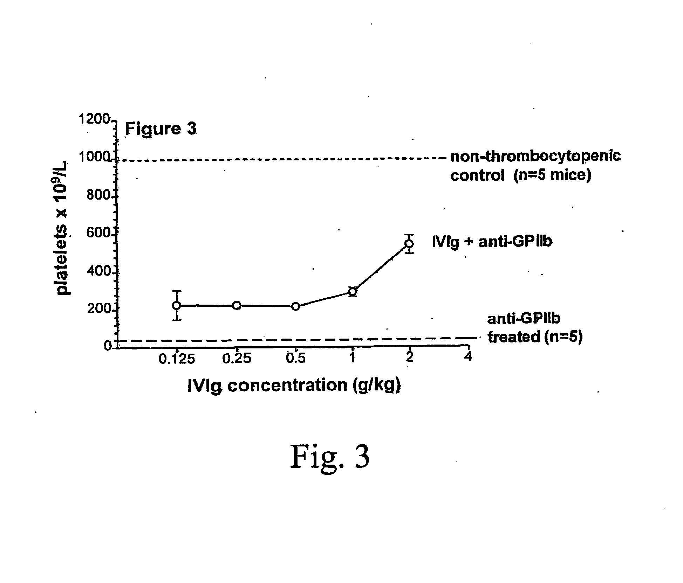

Currently, the standard dose of IVIG for treating ITP in humans is 2 g/kg body weight. In an independent experiment, the mice pretreated with as little as 0.12 g/Kg IVIG were partially protected from thrombocytopenia (Fig. 3).

In Fig. 3, the human IVIG was diluted in PBS, pH 7.2. The units on the x-axis are g IVIG / Kg mouse body weight. Each data point represents the mean in vivo platelet concentration (from whole blood) of 5 mice sampled 24 h post P-ITP induction.

### Platelet Analysis

Five (5) microlitres of whole blood was diluted 1/12,000 in a EDTA/PBS and acquired for 2 minutes on a flow-rate-calibrated FACscan flow cytometer (Becton-Dickinson, San Jose, CA). The number of gated platelets (as assessed by forward angle light scatter and side scatter) was used to calculate the platelet concentration. Reference samples were incubated with FITC-conjugated anti-mouse platelet antibody to ensure the proper platelet gate was set.

### Anti-idiotype depletion of IVIg

Protein G purified immunoglobulin from the sera of outbred CD1 strain mice (Cedarlane, Hornby, ON) was coupled to CNBr-activated Sepharose™ 4B. IVIG was depleted of mouse IgG-reactive components by incubation with 3 rounds of the IgG-coupled Sepharose using a batch method. ELISAs were performed essentially as described, plates were coated with a purified F(ab')₂ fragment of CD1 IgG, and IVIG vs. depleted IVIG were analyzed for CD1 F(ab')₂ reactivity. There was no detectable total protein loss after this IVIG manipulation. Data was analyzed using the unpaired Student's t test.

### Treatment of P-ITP mice with mIVIG

### Control mIVIG

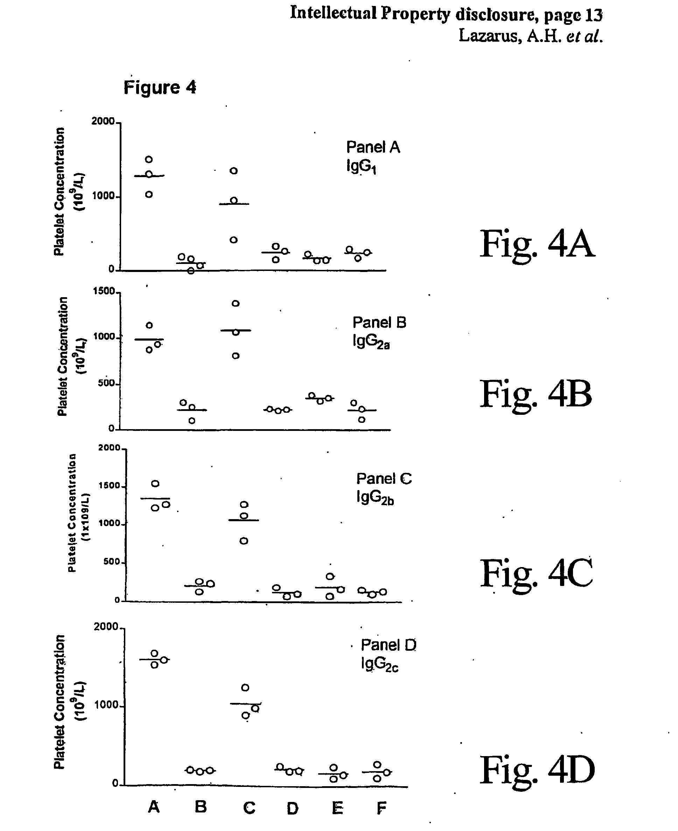

The mice injected with 2000 µg/Kg, 200 µg/Kg, or 20 µg/Kg of control mIVIG (i.e. mIVIG. without specificity to any relevant antigen in SCID mice) were not protected against thrombocytopenia (Fig. 4 A, columns D-F (clone Al 10-1), Fig. 4B, columns D-F (clone A110-2), Fig. 4C, columns D-F (clone A95-1) and Fig. 4D, columns D-F (clone A23-1). In comparison, mice therapeutically treated with a standard commercial human IVIG were protected against P-ITP (Figs. 4A to 4D, column C). The mIVIG's were all rat monoclonal antibodies and were purchased as "purified antibodies" from Pharmingen, unless otherwise stated.

As can be seen in Figs. 4A to 4D, monoclonal IVIG (mIVIG) (of irrelevant specificity) does not ameliorate P-ITP. In Figs. 4A to 4D, Column A represents unmanipulated mice, column B represents mice injected with anti-GPIIb, column C represents mice injected with 2 g IVIG /Kg body weight. Column D, mice injected with 2000 µg mIVIG /Kg body weight. Column E, mice injected with 200 µg mIVIG /Kg body weight. Column F, mice injected with 20 µg mIVIG /Kg body weight. Each data point represents the in vivo platelet concentration (from whole blood) of 1 mouse sampled 24 h post P-ITP induction.

### mIVIG specific for erythrocytes

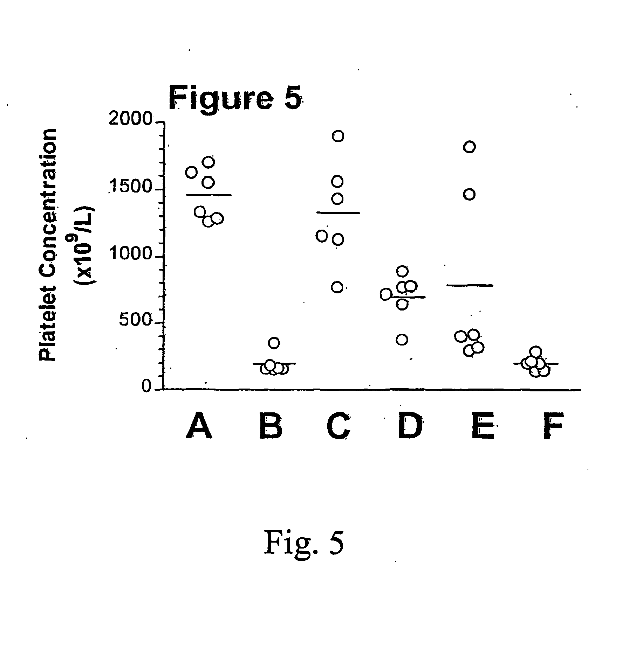

Mice injected with 2000 µg/Kg mIVIG with specificity for the CD24 antigen on erythrocytes (clone MI/69) were protected against P-ITP (Fig. 5, column D) while those treated with 200 µg/Kg were also somewhat protected but with more variable results (Fig. 5, column E). Mice injected with 20 µg/Kg mIVIG with specificity for the CD24 antigen were not protected against P-ITP (Fig. 5, column F). Mice therapeutically treated with a standard commercial human IVIG were protected against P-ITP in this experiment (Fig. 5 column C).

In Fig. 5, column A represents unmanipulated mice, columns B-F represent mice injected with anti-GPIIb, from which column C represents mice injected with 2 g IVIG /Kg body weight, column D represents mice injected with 2000 µg anti-CD24 /Kg body weight, column E represents mice injected with 200 µg anti-CD24 /Kg body weight, and column F represents mice injected with 20 µg anti-CD24 /Kg body weight.

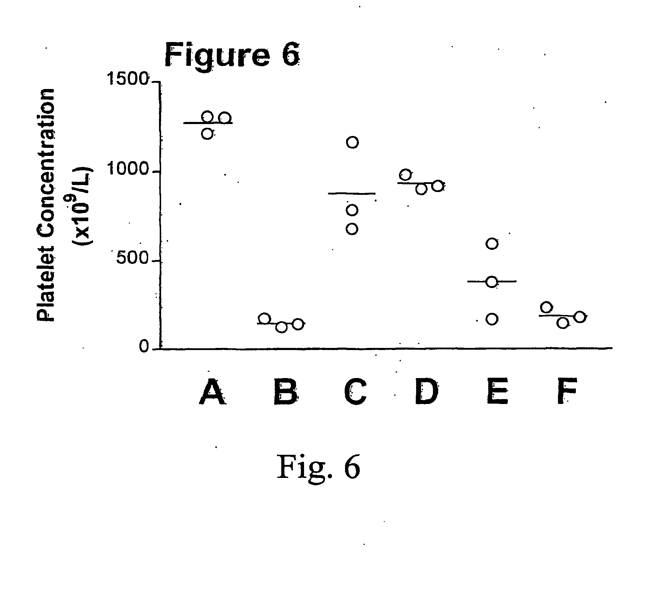

Mice injected with 2000 µg/Kg mIVIG with specificity for the TER-119 antigen (also known as the Ly-76 antigen) expressed on erythrocytes (clone TER-119) were protected against P-ITP (Fig. 6, column D). The degree of protection was comparable to 2 g IVIG /Kg body weight (Fig. 6, column C). Mice injected with 200 µg/Kg mIVIG with specificity for the TER-119 antigen were minimally protected against P-ITP (Fig. 6, column E). Mice injected with 20 µg/Kg mIVIG with specificity for the TER- 119 antigen were not protected against P-ITP (Fig. 6, column F).

In Fig. 6, column A represents unmanipulated mice, whereas columns B-F represent mice injected with anti-GPIIb, from which column C represents mice injected with 2 g IVIG /Kg body weight, column D represents mice injected with 2000 µg anti-TER-119 /Kg body weight, column E represents mice injected with 200 µg anti-TER-119 /Kg body weight, and column F represents mice injected with 20 µg anti-TER-119/Kg body weight.

### mIVIG which functionally block the Fcγ receptor RII & RIII

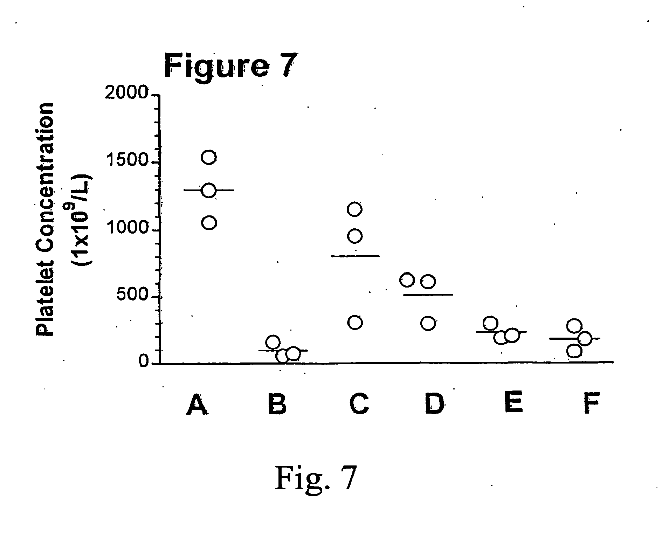

As mentioned previously, to determine if direct blockade of receptors in the reticuloendothelial system (RES) is a likely mechanism by which mIVIG may achieve its therapeutic effect, an mIVIG which specifically binds to and directly blocks the Fcγ receptors in the RES (Fig. 7) was examined. This mIVIG binds to the active site on the FcγRII (CD32) & RIII (CD 16) receptors and prevents Fcγ receptor-dependent platelet clearance. Mice injected with 2000 µg/Kg mIVIG with this mIVIG (clone 2.462) were partially protected against P-ITP (Fig. 7, column D) while those treated with 200 µg/Kg or 20 µg/Kg (Fig. 7, columns E and F) were not noticeably protected.

In Fig. 7, column A represents unmanipulated mice and columns B-F represent mice injected with anti-GPIIb, from which column C represents mice injected with 2 g IVIG /Kg body weight, column D represents mice injected with 2000 µg anti-CD16+32 /Kg body weight, column E represents mice injected with 200 µg anti-CD16+32 /Kg body weight, and column F represents mice injected with 20 µg anti-CD16+32/Kg body weight.

### mIVIG which bind to a non-erythrocyte antigen

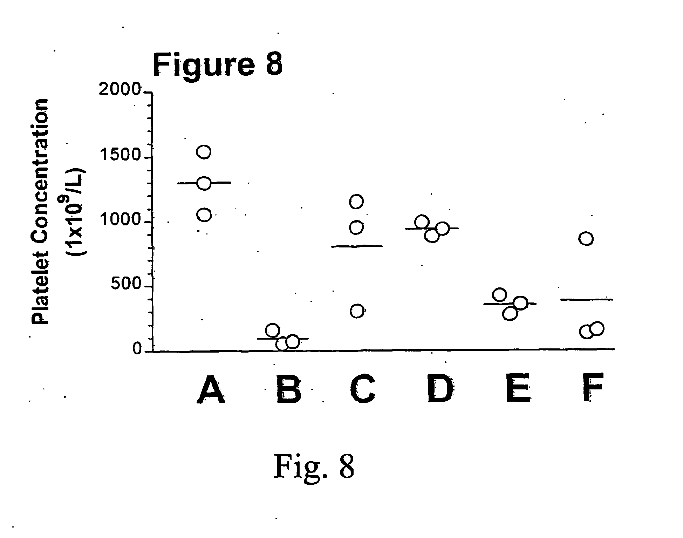

Also as mentioned previously, to demonstrate that mIVIG directed against non-erythroid cells can act as a therapeutic agent capable of reversing thrombocytopenia, an mIVIG directed to the CD44 antigen (Fig. 8) was evaluated. The CD44 antigen is expressed on most cells but not on erythrocytes. Mice injected with 2000 µg/Kg mIVIG with specificity for the CD44 antigen (clone LOU /MN) were protected against P-ITP (Fig. 8, column D) while those treated with 200 µg/Kg gave a much lower effect (Fig. 8, column E) . Mice injected with 20 µg/Kg of this mIVIG gave variable results (Fig. 8, column E).

In Fig. 8, column A represents unmanipulated mice, whereas columns B-F represent mice injected with anti-GPIIb, from which column C represent mice injected with 2 g IVIG /Kg body weight, column D represents mice injected with 2000 µg anti-CD44 /Kg body weight, column E represents mice injected with 200 µg anti-CD44 /Kg body weight, and column F represents mice injected with 20 µg anti-CD44 /Kg body weight.

The effective dose of the mIVIGs used herein is 3 log fold lower than standard (polyclonal) IVIG. mIVIG therapy is anticipated to be less expensive than human IVIG, mIVIG can be manufactured by recombinant means, and would be available in unlimited supply. Also, whereas the mechanism of action of standard IVIG is controversial, the mechanism of action of mIVIG is described.

The major site of platelet destruction in ITP is the spleen. The spleen has the capacity to remove antibody-opsonized cells via the activity of monocytes and macrophages (reticuloendothelial system, RES). To determine if mIVIG which can successfully ameliorate thrombocytopenia also blocks the RES, RES blockade experiments were performed with mice treated with the indicated mIVIGs. It was observed that the anti-LY76 and the anti-CD24 (clone MI/69), (which both ameliorate thrombocytopenia) both significantly blocked RES function. The antibodies blocked RES function as well as IVIG. Albumin was used as a negative control (does not affect thrombocytopenia and is not expected to block RES function) and albumin treated mice had the highest erythrocyte clearance rate, as expected.

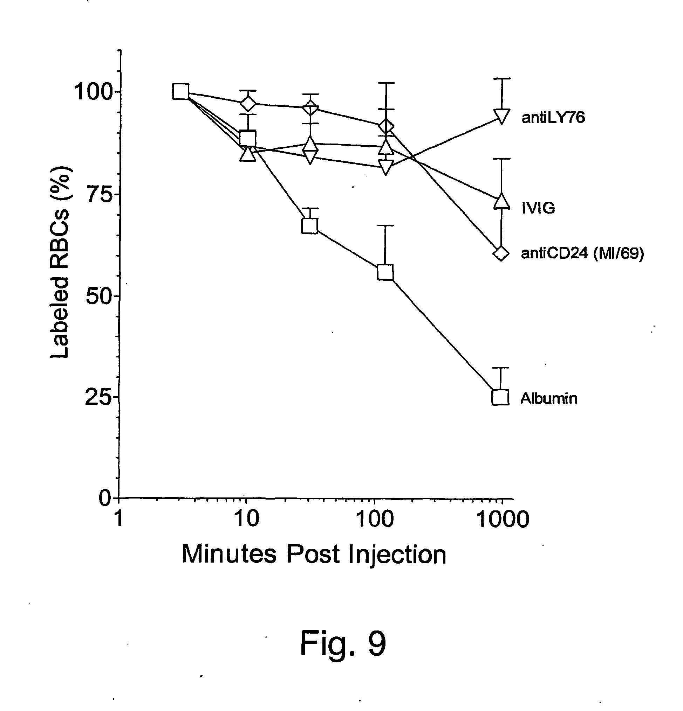

In Fig. 9, SCID mice were individually injected intravenously with mIVIG, either 50 µg (200µl) of anti-LY76, or 50 µg anti-CD24 (clone MI/69), or intraperitoneally with 50 mg (1ml) of IVIG or intraperitoneally with 50 mg of human serum albumin. After 24 hours, all mice were given an intravenous injection of 200µl of PKH26 labeled, antibody sensitized SCID mouse erythrocytes. Blood was removed from each mouse at the indicated time points and the circulating erythrocytes were assessed by flow cytometry.

The PKH26 red fluorescent cell linker kit was purchased from Sigma (St. Louis, MO). Two mls of whole blood was obtained from 10-20 separate SCID mice. This blood was centrifuged at 2,000xg for 15 min to obtain 1 ml of packed erythrocytes. These packed erythrocytes were resuspended in 4 ml of PBS and incubated with 10 µg of anti-mouse Ly-76 antibody at 22°C for 0.5 h (to opsonize the erythrocytes). The opsonized erythrocytes were then washed twice with PBS and labeled with a fluorescent marker (PKH 26, from Sigma, St. Louis MO) as follows; the opsonized erythrocytes were resuspended in 3 ml of PKH 26 'diluent C' (Sigma, St. Louis MO) and mixed with another 4 ml of 'diluent C' containing 10 µl of the 'PKH 26 linker'. After 5 minutes of incubation at 22°C with constant shaking, the mixture was incubated with an equal volume of PBS containing 1% bovine serum albumin for 5 minutes. The erythrocytes were washed 5 times (40 ml PBS/wash) and resuspended In 2 ml PBS. Mice were then injected via the tail vein with 200 µl of these labeled cells (50% packed erythrocytes). After 3 min, 10 min, 30 min, 120 min, and 960 min each mouse was bled (25 ul/bleed) vial the tail vein and the number of total erythrocytes as well as the number of PKH 26-fluorescent erythrocytes were enumerated by flow cytometry. The % of labeled erythrocytes at the 3 min time point was considered to be 100%.

## Claims

1. A pharmaceutical composition for use in treating thrombocytopenia in a mammal, comprising an effective amount of a monoclonal antibody specific for blood cells in combination with a pharmaceutically acceptable carrier, wherein the monoclonal antibody is an anti-red blood cell antibody and is capable of inhibiting Reticuloendothelial System (RES).

2. The pharmaceutical composition of claim 1, wherein the anti-red blood cell antibody is anti-CD24 or anti-TER-119.

3. The pharmaceutical composition of any one of claims 1-2, further comprising human IVIG.

4. The pharmaceutical composition of any one of claims 1 to 3, wherein the monoclonal antibody therapeutically increases and maintains platelet counts.

5. The pharmaceutical composition of any one of claims 1 to 3, wherein the mammal is a human or an animal.

6. The pharmaceutical composition of any one of claims 1 to 3, wherein the antibody is adapted for intravenous, intraperitoneal, intramuscular or subcutaneous administration.

7. The pharmaceutical composition of any one of claims 1 to 3 being free of contaminants such as TGFβ.

8. Use of a pharmaceutical composition as defined in claim 1, comprising at least 1µg of the monoclonal antibody per kg of body weight of the mammal, in the manufacture of a medicament for increasing the level of platelets in a mammal experiencing thrombocytopenia.

9. A pharmaceutical composition as defined in claim 1, comprising at least 1µg of the monoclonal antibody per kg of body weight of the mammal, for increasing the level of platelets in a mammal experiencing thrombocytopenia.

## Patentansprüche

1. Pharmazeutische Zusammensetzung für die Verwendung bei der Behandlung von Thrombozytopenie bei einem Säugetier, umfassend eine wirksame Menge eines monoklonalen Antikörpers, der für Blutzellen spezifisch ist, in Kombination mit einem pharmazeutisch verträglichen Träger, wobei der monoklonale Antikörper ein Antikörper gegen rote Blutzellen ist und dazu in der Lage ist, das Retikuloendotheliale System (RES) zu hemmen.

2. Pharmazeutische Zusammensetzung nach Anspruch 1, wobei der Antikörper gegen die roten Blutzellen ein Anti-CD24 oder Anti-TER-119 ist.

3. Pharmazeutische Zusammensetzung nach einem der Ansprüche 1 bis 2, welche weiterhin humanes IVIG umfasst.

4. Pharmazeutische Zusammensetzung nach einem der Ansprüche 1 bis 3, wobei der monoklonale Antikörper die Blutplättchenzahl therapeutisch erhöht und aufrecht erhält.

5. Pharmazeutische Zusammensetzung nach einem der Ansprüche 1 bis 3, wobei das Säugetier ein Mensch oder ein Tier ist.

6. Pharmazeutische Zusammensetzung nach einem der Ansprüche 1 bis 3, wobei der Antikörper für die intravenöse, intraperitoneale, intramuskuläre oder subkutane Verabreichung angepasst ist.

7. Pharmazeutische Zusammensetzung nach einem der Ansprüche 1 bis 3, welche frei von Kontaminanten, wie z. B. TGFβ, ist.

8. Verwendung einer pharmazeutischen Zusammensetzung, wie sie in Anspruch 1 definiert ist, umfassend wenigstens 1µg des monoklonalen Antikörpers pro kg des Körpergewichts des Säugetiers, bei der Herstellung eines Arzneimittels zur Erhöhung des Bluttplättchenniveaus in einem Säugetier, welches unter Thrombozytopenie leidet.

9. Pharmazeutische Zusammensetzung nach Anspruch 1, umfassend wenigstens 1µg des monoklonalen Antikörpers pro kg des Körpergewichts des Säugetiers für die Erhöhung des Bluttplättchenniveaus in einem Säugetier, dass unter Thrombozytopenie leidet.

## Revendications

1. Composition pharmaceutique destinée à être utilisée dans le traitement de la thrombopénie chez un mammifère, comprenant une quantité efficace d'un anticorps monoclonal spécifique pour les cellules du sang en combinaison avec un vecteur pharmaceutiquement acceptable, où l'anticorps monoclonal est un anticorps anti-hématies et est capable d'inhiber le système réticulo-endothélial (SRE).

2. Composition pharmaceutique selon la revendication 1, où l'anticorps anti-hématies est anti-CD24 ou anti-TER-119.

3. Composition pharmaceutique selon l'une quelconque des revendications 1-2, comprenant en outre des IVIG humaines.

4. Composition pharmaceutique selon l'une quelconque des revendications 1 à 3, où l'anticorps monoclonal augmente et maintient thérapeutiquement les numérations plaquettaires.

5. Composition pharmaceutique selon l'une quelconque des revendications 1 à 3, où le mammifère est un humain ou un animal.

6. Composition pharmaceutique selon l'une quelconque des revendications 1 à 3, où l'anticorps est adapté pour l'administration intraveineuse, intrapéritonéale, intramusculaire ou sous-cutanée.

7. Composition pharmaceutique selon l'une quelconque des revendications 1 à 3 dépourvue de contaminants tels que TGFβ.

8. Utilisation d'une composition pharmaceutique telle que définie dans la revendication 1, comprenant au moins 1 µg de l'anticorps monoclonal par kg de poids corporel du mammifère, dans la fabrication d'un médicament pour augmenter le niveau de plaquettes chez un mammifère subissant une thrombopénie.

9. Composition pharmaceutique telle que définie dans la revendication 1, comprenant au moins 1 µg de l'anticorps monoclonal par kg de poids corporel du mammifère, pour augmenter le niveau de plaquettes chez un mammifère subissant une thrombopénie.
